# EUROPEAN PATENT APPLICATION

(11) **EP 4 439 437 A1**
(43) Date of publication of application: **02.10.2024**
(21) Application number: 22898560.2
(22) Date of filing: 22.11.2022
(51) Int. Cl.: G06Q 30/0601, G16H 50/30

(54) **INFORMATION PROCESSING SYSTEM**

(30) Priority: 25.11.2021 JP 2021191085
(71) Applicant: Kao Corporation, Tokyo 103-8210 (JP)
(72) Inventor: NIWANO, Yu, Tokyo 131-8501 (JP); KIKUCHI, Sho, Tokyo 131-8501 (JP)
(74) Representative: Hoffmann Eitle
(86) International application number: PCT/JP2022/043137
(87) International publication number: WO 2023/095777

(57) **Abstract**

An information processing system is an information processing system that provides information regarding a product for skin to each user, including: a control unit. The control unit analyzes pieces of biological information regarding skin of a plurality of users provided from the plurality of users and generates a plurality of skin classifications for classifying the skin of the plurality of users on the basis of an analysis result. Further, the control unit stores user identification information for identifying a user of a source of the biological information in association with one of the generates skin classifications and stores product information regarding a plurality of products relating to skin in association with the corresponding skin classification. Further, the control unit receives, from a user terminal of a user corresponding to the user identification information, a search request for product information using the skin classification as a search criterion. Then, the control unit searches for the product information corresponding to the skin classification used as the search criterion in accordance with the search request and transmits the searched product information to the user terminal.

## Description

### Technical Field

The present invention relates to an information processing system that provides information regarding a product relating to skin for each user.

### Background Art

In the past, there has been a technology for providing information regarding products relating to skin, such as cosmetics, in accordance with a user. For example, the following Patent Literature 1 discloses a method of providing a system for selecting a treatment cosmetic product that suits the consumer's skin condition, including: (a) collecting information regarding a plurality of features relating to the skin condition from the consumer; (b) inputting information to a computerized knowledge system that selects a cosmetic product likely to be effective for the consumer from at least two types of cosmetic products effective for the skin condition; and (c) providing a treatment cosmetic product for treating the skin condition to the consumer, the computerized knowledge system being obtained from a correlation between responses of a population of individuals to a plurality of cosmetic products and features measured for the individuals before using the plurality of cosmetic products.

### Citation List

### Patent Literature

Patent Literature 1: Japanese Patent Application Laid-open No. 2003-260031

### Disclosure of Invention

### Technical Problem

However, the above Patent Literature 1 does not disclose any technology for users to search for and find a product that suits their skin.

An object of the present invention is to allow users to easily search for information regarding products that objectively correspond to the user's skin concerns and interests.

### Solution to Problem

An information processing system according to an embodiment of the present invention is a system that provides information regarding a product for skin to each user, including: a control unit. The control unit analyzes pieces of biological information regarding skin of a plurality of users provided from the plurality of users and generates a plurality of skin classifications for classifying the skin of the plurality of users on a basis of an analysis result. Further, the control unit stores user identification information for identifying a user of a source of the biological information in association with one of the generated skin classifications and stores product information regarding a plurality of products relating to skin in association with the corresponding skin classification. Further, the control unit receives, from a user terminal of a user corresponding to the user identification information, a search request for product information using the skin classification as a search criterion. Then, the control unit searches for the product information corresponding to the skin classification used as the search criterion in accordance with the search request and transmits the searched product information to the user terminal. Advantageous Effects of Invention

In accordance with the information processing system according to an embodiment of the present invention, it is possible to allow users to easily search for information regarding products that objectively correspond to the user's skin concerns and interests.

### Brief Description of Drawings

[Fig. 1] Fig. 1 is a diagram showing a configuration of a cosmetics information providing system according to an embodiment of the present invention.
[Fig. 2] Fig. 2 is a diagram showing a hardware configuration of a cosmetics information providing server according to the embodiment of the present invention.
[Fig. 3] Fig. 3 is a diagram showing a configuration of a database included in the cosmetics information providing server according to the embodiment of the present invention.
[Fig. 4] Fig. 4 is a flowchart showing flow of skin classification generation processing by the cosmetics information providing server according to the embodiment of the present invention.
[Fig. 5] Fig. 5 is a diagram showing an example of RNA information to be analyzed by the cosmetics information providing server according to the embodiment of the present invention for generating a skin classification.
[Fig. 6] Fig. 6 is a diagram showing an example of the skin classification generated by the cosmetics information providing server according to the embodiment of the present invention.
[Fig. 7] Fig. 7 is a diagram showing another example of the skin classification generated by the cosmetics information providing server according to the embodiment of the present invention.
[Fig. 8] Fig. 8 is a flowchart showing flow of cosmetics information providing processing by the cosmetics information providing server according to the embodiment of the present invention.
[Fig. 9A] Fig. 9A is a diagram showing an example of a search result page that is generated by the cosmetics information providing server according to the embodiment of the present invention and displayed on a user terminal.
[Fig. 9B] Fig. 9B is a diagram showing an example of a search result page that is generated by the cosmetics information providing server according to the embodiment of the present invention and displayed on a user terminal.
[Fig. 9C] Fig. 9C is a diagram showing an example of a search result page that is generated by the cosmetics information providing server according to the embodiment of the present invention and displayed on a user terminal.

### Mode(s) for Carrying Out the Invention

An embodiment of the present invention will be described below with reference to the drawings. In this embodiment, cosmetics will be given as an example of a product.

### [Configuration of system]

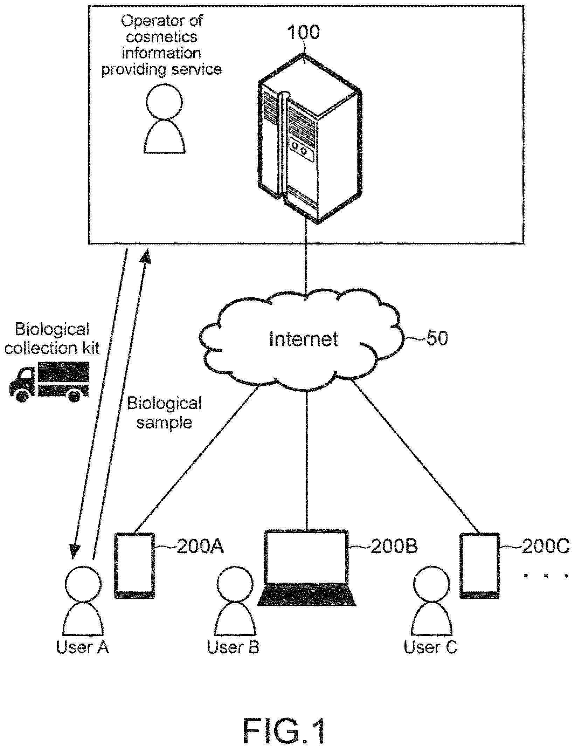

As shown in Fig. 1, this system includes a cosmetics information providing server 100 on the Internet 50 and a plurality of user terminals 200.

The cosmetics information providing server 100 is a web server (information processing apparatus) operated by an operator of a portal site (a cosmetics information providing site) where information regarding cosmetics (including external preparations such as quasi-drugs) can be searched for. The cosmetics information providing server 100 is connected to the plurality of user terminals 200 through the Internet 50.

The cosmetics information providing server 100 provides a cosmetics information providing service to a user of the user terminal 200 on the above portal site. Specifically, the cosmetics information providing server 100 searches for cosmetics information matching the search criterion on the basis of a search request from the user terminal 200, generates a web page containing the search result, and transmits the generated web page to the user terminal 200.

The user terminals 200 (200A, 200B, 200C ...) are terminals used by users, such as smartphones, mobile phones, tablet PCs (Personal Computers), notebook PCs, and desktop PCs. The user terminal 200 accesses the cosmetics information providing server 100, receives the above web page, and displays the received web page on a screen using a browser or the like.

The user terminal 200 determines a search criterion for cosmetics on the basis of the user's operation and transmits a search request for cosmetics information based on the search criterion to the cosmetics information providing server 100.

In this embodiment, the cosmetics information providing server 100 generates, on the basis of biological information provided from the user of the user terminal 200, a skin classification for classifying the skin of each user, receives a search request using the skin classification as a search criterion from the user terminal 200, and provides the search result to the user terminal 200 in response thereto.

In order to generate the skin classification, the service operator sends a biological collection kit to each user, and the user returns a biological sample collected using the kit to the operator. In this embodiment, the biological information is, for example, RNA information, and the biological collection kit is a tool for collecting biological samples such as sebum, stratum corneum, saliva, urine, and blood from each user. Further, the operator includes those entrusted by the operator.

Data measured from the biological sample is input to the cosmetics information providing server 100, and the cosmetics information providing server 100 generates a skin classification on the basis of the data. Details of processing of generating the skin classification and processing of providing cosmetics information using the skin classification will be described below.

### [Hardware configuration of cosmetics information providing server]

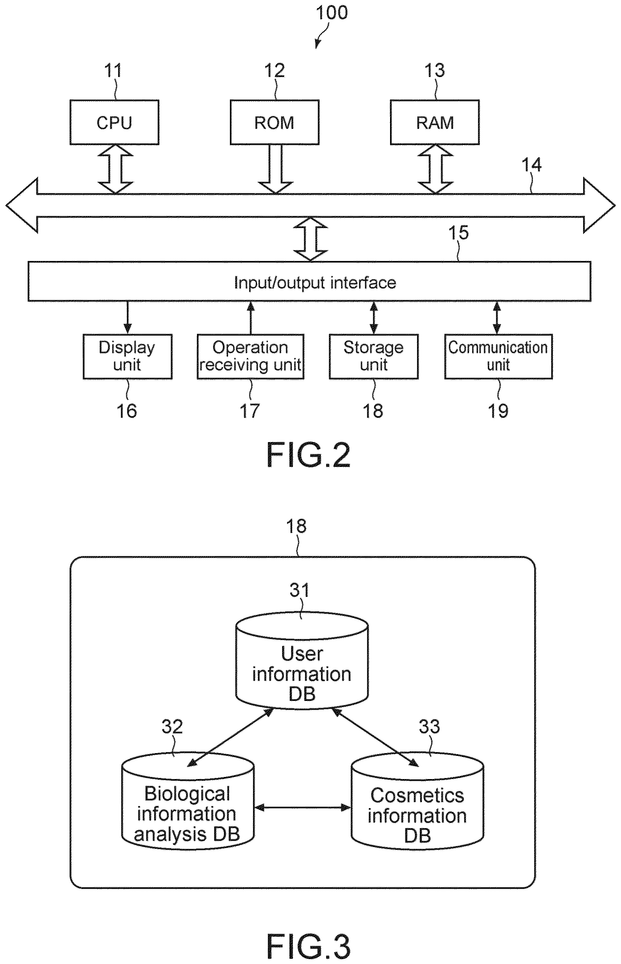

As shown in Fig. 2, the cosmetics information providing server 100 includes a CPU (Central Processing Unit) 11, a ROM (Read Only Memory) 12, a R_AM (Random Access Memory) 13, an input/output interface 15, and a bus 14 that connects these to each other.

The CPU 11 accesses the R_AM 13 and the like as necessary, and integrally controls the respective blocks of the cosmetics information providing server 100 while performing various types of arithmetic processing. The ROM 12 is a non-volatile memory in which an OS to be executed by the CPU 11 and firmware such as programs and various parameters are statically stored. The R_AM 13 is used as a work area for the CPU 11 and temporarily stores the OS, various applications being executed, and various types of data being processed.

A display unit 16, an operation receiving unit 17, a storage unit 18, a communication unit 19, and the like are connected to the input/output interface 15.

The display unit 16 is, for example, a display device using LCD (Liquid Crystal Display), OELD (Organic ElectroLuminescence Display), CRT (Cathode Ray Tube), or the like.

The operation receiving unit 17 is, for example, a pointing device such as a mouse, a keyboard, a touch panel, or another input device. In the case where the operation receiving unit 17 is a touch panel, the touch panel can be integrated with the display unit 16.

The storage unit 18 is a non-volatile memory such as an HDD (Hard Disk Drive), a flash memory (SSD; Solid State Drive), and another solid-state memory. The storage unit 18 stores the above OS, various applications, and various types of data.

As will be described below, particularly in this embodiment, the storage unit 18 includes a user information database, a biological information analysis database, and a cosmetics information database in addition to a program such as an application necessary for skin classification generation processing and cosmetics information providing processing described below.

The communication unit 19 is, for example, a NIC (Network Interface Card) for Ethernet or various modules for wireless communication such as a wireless LAN, and is responsible for communication processing with the above user terminal 200.

Note that although not shown, the basic hardware configuration of the user terminal 200 is also substantially the same as the hardware configuration of the above cosmetics information providing server 100.

### [Database configuration of cosmetics information providing server]

As shown in Fig. 3, the cosmetics information providing server 100 includes a user information database 31, a biological information analysis database 32, and a cosmetics information database 33 in the storage unit 18. Note that the respective databases may be stored not in the storage unit 18 but in a storage device or a server externally connected to the cosmetics information providing server 100.

The user information database 31 stores attribute information regarding a user who is the source of biological information for each user. Examples of the attribute information regarding a user include information regarding the user's skin, such as skin quality that he/she perceives or understands, the user's preference of cosmetics, and skin problems that he/she has, in addition to general information such as a name, a user ID for identifying a user, an age (an age group), an occupation, an address (residence area), a gender, and an e-mail address. Note that the user ID functions as account information for using this cosmetics information providing service. It is favorable that the attribute information regarding a user can be selected from a plurality of options. For example, in the case of skin quality, the attribute information may be selected from a plurality of viewpoints such as the level of sensitive skin and skin color in addition to the classifications of dry skin and oily skin.

Further, the user information database 31 also stores, for each user, information indicating the skin classification into which each user is classified, of the skin classifications generated by skin classification generation processing described below.

The biological information analysis database 32 stores RNA information measured from the biological sample collected from each user and the above information regarding the skin classification generated on the basis of the RNA information. The RNA information is specifically data obtained by extracting and preparing RNA from each biological sample, generating cDNA by reverse transcription, and then measuring the expression level of each RNA species by sequencing, PCR, or the like. For the measurement analysis of RNA information from the biological sample in this case, known devices, means, and algorithms can be used. Further, examples of the biological sample include sebum, stratum corneum, saliva, urine, and blood, and sebum is favorable from the viewpoint of convenience and being able to identify skin surface portions.

The cosmetics information database 33 (product information DB 33) stores information regarding cosmetics, i.e., information such as a manufacturer name of each cosmetic, a product name (brand name), categories of cosmetics (e.g., beauty serum, lotion, cleansing agent, face wash, pack, cream, etc.), and expected effects (e.g., brightening, pore care, acne care, exfoliation care, skin firmness, sensitive skin care, moisture, anti-aging, gloss, natural makeup, waterproof, color retention, color development, etc.).

Further, the cosmetics information database 33 also stores product evaluation information indicating user evaluation of each cosmetic. Examples of the product evaluation information include an average value of values (quantitative evaluation information) evaluated by users on the respective cosmetics using scores or multiple levels (e.g., 5 levels or a perfect score of 100), for each product. Further, examples of the product evaluation information to be input include text information (qualitative evaluation information) indicating a user review (comment) for each cosmetic. The qualitative evaluation information is converted into quantitative evaluation information using a predetermined algorithm (e.g., quantifying each review in accordance with the ratio of positive and negative phrases in the text), and the average value thereof is calculated. Further, examples of the product evaluation information also include the user ID of the user who is the evaluation source.

The cosmetics information is associated with the above skin classification information and attribute information via the user ID of the user who is the evaluation source of the above evaluation information.

These databases are mutually referenced and used as necessary in skin classification generation processing and cosmetics information providing processing described below by the cosmetics information providing server 100.

### [Operation of cosmetics information providing server]

Next, an operation of the cosmetics information providing server 100 configured as described above will be described. The operation is executed in cooperation with hardware such as the CPU 11 and the communication unit 19 of the cosmetics information providing server 100 and software stored in the storage unit 18. In the following description, for convenience, the CPU 11 is assumed to be the main operating body.

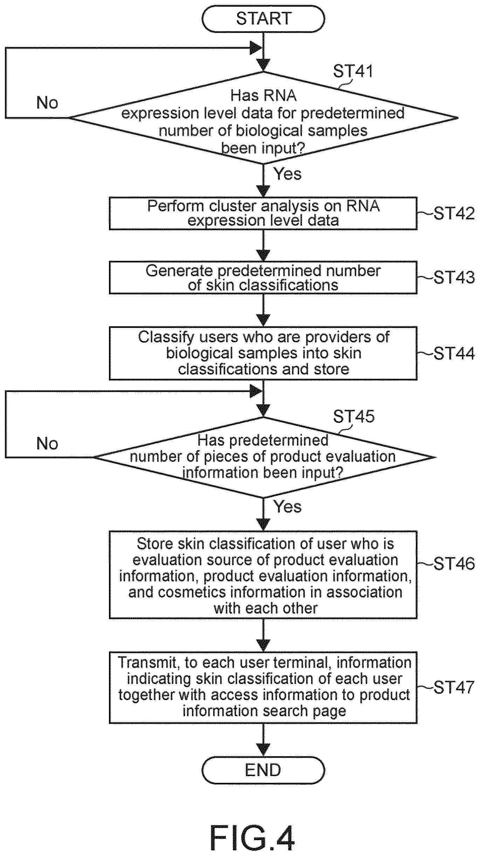

First, skin classification generation processing by the cosmetics information providing server 100 will be described. As shown in Fig. 4, the CPU 11 of the cosmetics information providing server 100 determines whether or not RNA expression level data for a predetermined number of biological samples has been input (Step 41). Here, the predetermined number of samples is based on, for example, a predetermined number of people, such as 200 or more, 500 or more, and 1000 or more.

In the case where it is determined that RNA expression level data for a predetermined number of samples has been input (Yes in Step 41) or it is determined that the regularly set timing has arrived, the CPU 11 performs cluster analysis on the RNA expression level data (Step 42).

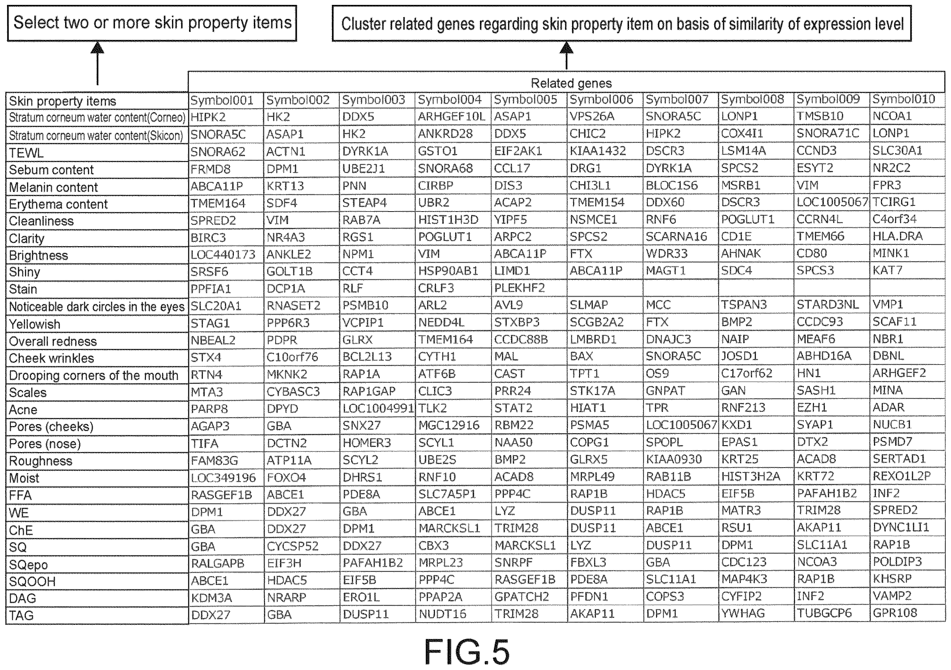

Specifically, as shown in Fig. 5, the CPU 11 selects two or more skin property items from a plurality of skin property items for evaluating the physical properties of the user's skin (e.g., 30 items in the figure, including stratum corneum water content, TEWL (transepidermal water loss), sebum content, melanin content, erythema content, overall redness, etc.). Note that Fig. 5 lists 10 genes with high correlation as characteristic genes on the basis of the relationship between the RNA expression level and evaluation or measurement results regarding various skin properties, and information that has already been obtained can be used (see Japanese Patent Application Laid-open No. 2020-74769).

Then, the CPU 11 clusters the related genes of each sample regarding the selected skin property item on the basis of the similarity of the expression level (expression pattern). As the clustering method, either hierarchical or non-hierarchical clustering may be used.

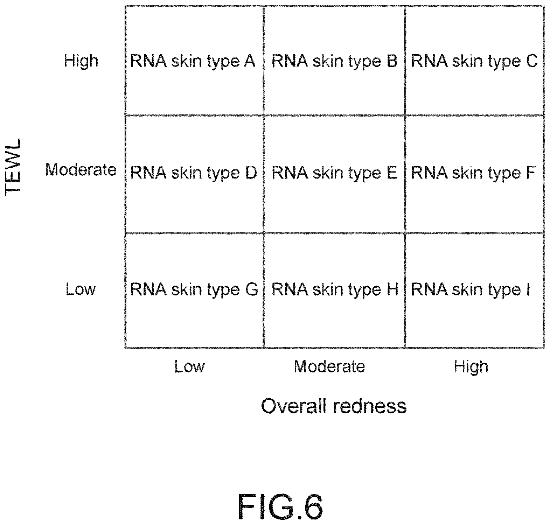

Subsequently, the CPU 11 generates a predetermined number of skin classifications according to the number of clusters classified by the clustering (Step 43). As shown in Fig. 6, in this embodiment, for example, TEWL and the overall redness are selected as skin property items, and the similarity of gene expression levels in both items is divided into three patterns (high, moderate, and low) to generate a total of nine skin classifications (RNA skin types A to I). However, the number of skin property items to be selected is not limited to two, and the number of classifications of the similarity of the gene expression level is also not limited to three. Further, a plurality of types of skin classifications may be generated by generating a plurality of different combinations of a plurality of skin property items. For example, RNA (gene) may be selected on the basis of the skin property items that suit various skin concerns such as age spots and wrinkles. In addition, clustering may be performed by combining proteome information and information regarding skin surface bacteria in addition to RNA information.

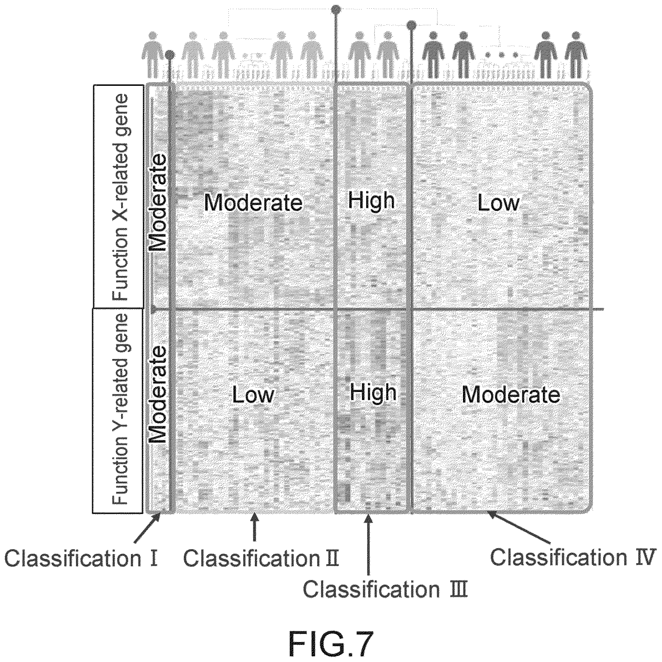

The clustering performed by the CPU 11 includes a skin classification based on 1000 or more and 20000 or less pieces of RNA information as the number of gene species other than a skin classification based on a specific skin property item. As shown in Fig. 7, for example, by performing clustering on the basis of the expression levels of the related gene of a certain function (function X) and the related gene of another function (function Y), four skin classifications, i.e., a classification I (function X: moderate expression level; function Y: moderate expression level), a classification II (function X: moderate expression level; function Y: low expression level), a classification III (function X: high expression level; function Y: high expression level), and a classification IV (function X: low expression level; function Y: moderate expression level). That is, in the case where the RNA profiles of users are similar, it can be presumed that they also have similar skin concerns or similar effects of cosmetics. Therefore, it is possible to generate a skin classification by using only the similarity of gene information (RNA expression level) without being associated with a specific skin property item.

Subsequently, the CPU 11 classifies each user who is the source of the biological sample into the above skin classification and stores the information indicating the skin classification of each user in the above user information database 31 in association with the user ID (Step 44).

Subsequently, the CPU 11 determines whether or not a predetermined number of pieces of product evaluation information has been input for each category of cosmetics (Step45). The predetermined number is, for example, approximately several hundred, but is not limited thereto. Further, when each user applies for delivery of a biological collection kit to the user on, for example, the above cosmetics information providing site, the application form may include a questionnaire screen for inputting the above product evaluation information and the product evaluation information may be transmitted to the cosmetics information providing server 100 via the questionnaire screen.

Further, the application form may also include an input field for the user's attribute information (general information and information regarding user's skin) on the above user information database 31 and the attribute information may be transmitted to the cosmetics information providing server 100 via the application form.

Further, each time the evaluation information is added for each product, the product evaluation information is calculated as an average value of numerical values indicated by the information and stored.

In the case where it is determined that one or a predetermined number of pieces of product evaluation information has been input for each cosmetics category (Yes in Step 45), the CPU 11 stores the skin classification of the user who is the evaluation source of the product evaluation information, the product evaluation information, and the cosmetics information in association with each other (Step 46).

That is, the CPU 11 stores each piece of information while which skin classification the user who is the evaluation source of the product evaluation information for certain cosmetics information is classified into, or what kind of cosmetic a user classified into a certain skin classification evaluates and how he/she evaluates the cosmetic, can be searched for on the basis of the user ID included in the product evaluation information and the skin classification corresponding to the user ID. In other words, the cosmetics information is associated with the skin classification on the basis of the product evaluation information.

Then, the CPU 11 transmits, to the user terminal 200 of the source of each biological sample, the information indicating the skin classification of each user by, for example, e-mail, various messenger applications, or the notification function on the above cosmetics information providing site (Step 47).

At this time, the CPU 11 may transmit access information (URL, a two-dimensional barcode, or the like) to the cosmetics information search page on the cosmetics information providing site together with the skin classification information. This allows the user not only to understand his/her skin classification but also to access the search page and obtain cosmetics information that suits the classification as soon as he/she knows the skin classification.

Next, cosmetics information providing processing by the cosmetics information providing server 100 using the above skin classification as a search criterion will be described.

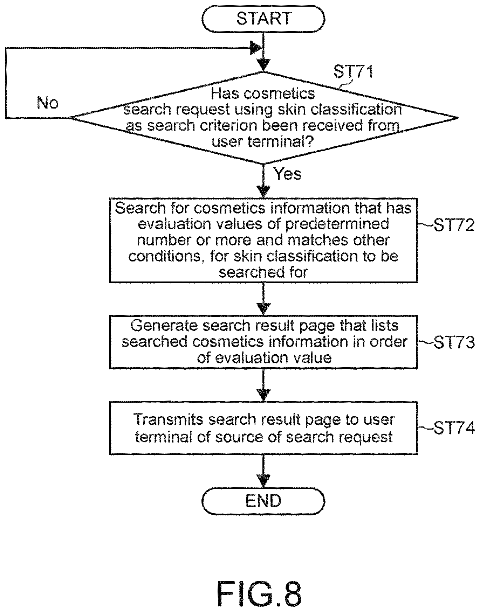

As shown in Fig. 8, the CPU 11 determines whether or not a cosmetics search request using a skin classification as a search criterion has been received from the user terminal 200 via the above cosmetics information search page (Step 71). The cosmetics search request may include a search criterion (e.g., a product category or a user attribute) other than the skin classification as an AND condition.

In the case where it is determined that the above search request has been received (Yes in Step 71), the CPU 11 searches for cosmetics information that has top evaluation values of a top predetermined number (e.g., top three or five) and matches other conditions, for the skin classification to be searched for (Step 72) .

That is, the CPU 11 extracts cosmetics information that matches the skin classification used as the search criterion and has evaluations values of the top predetermined number given by a user group that matches the user attribute used as the other search criteria, of pieces of cosmetics information of the product category used as the search criterion on the cosmetics information database 33.

Subsequently, the CPU 11 generates a search result page that lists the searched cosmetics information in order of evaluation value (in a ranking format) (Step73).

Then, the CPU 11 transmits the search result page to the user terminal 200 of the source of the search request (Step 74).

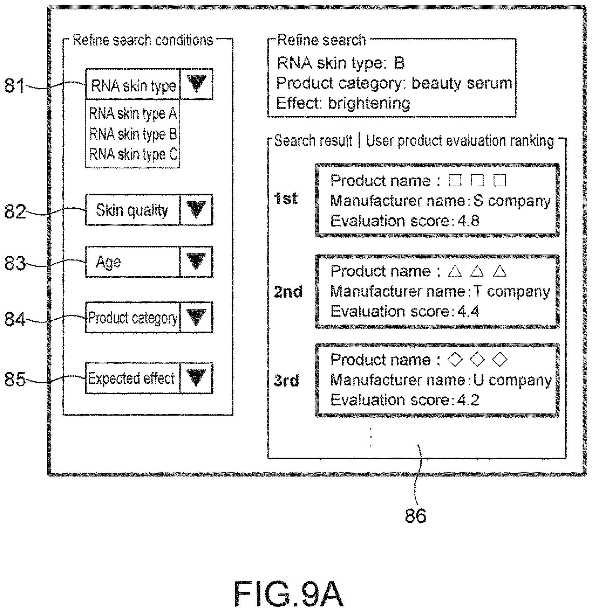

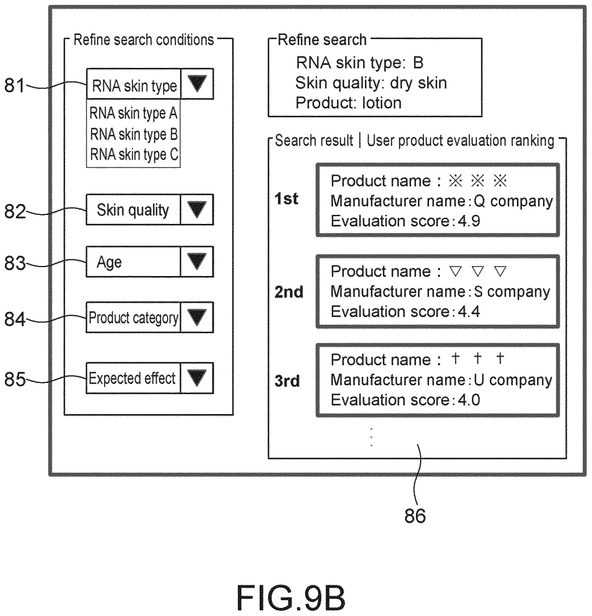

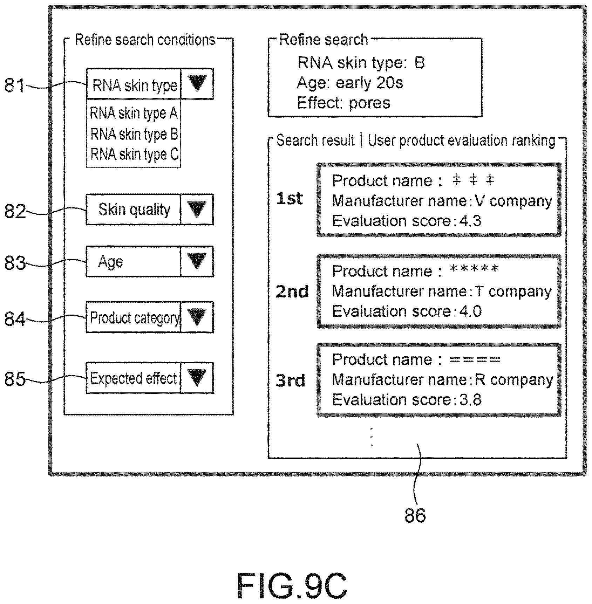

As shown in Fig. 9A, Fig. 9B, and Fig. 9C, on the search page, a skin quality selection menu 82, an age selection menu 83, a product category selection menu 84, and an effect selection menu 85 in which skin quality, an age, a product category, and an expected effect can be respectively selected as search criteria are provided in addition to a skin classification selection menu 81 in which a skin classification (RNA skin type) can be selected as a search criterion by, for example, a pull-down menu.

The user of the user terminal 200 selects search criteria using these menus and transmits a search request to the cosmetics information providing server 100 by a search request transmission button (not shown) or the like, thereby obtaining a cosmetics evaluation ranking 86 as shown in the figures.

As shown in the figures, it can be seen that cosmetics information to be displayed differs depending on the skin classification. Examples of the cosmetics information to be displayed included, but not limited to, information such as a product name, a manufacturer name, and an evaluation value.

As described above, according to this embodiment, the cosmetics information providing server 100 allows users to easily search for cosmetics information that objectively corresponds to the user's skin concerns and interests on the basis of objective data that is a skin classification generated on the basis of a biological sample. In addition thereto, by providing cosmetics information also taking into account user's attribute information regarding cosmetics and subjective evaluation information of other users, it is possible to give the user a sense of satisfaction and relief.

### [Modified example]

Although an embodiment of the present invention has been described above, it goes without saying that the present invention is not limited to only the above-mentioned embodiment and various modifications can be made without departing from the essence of the present invention.

Although RNA information has been used as biological information to be analyzed in the above embodiment, the proteome (protein) collected from the user's stratum corneum, sebum, or the like may be analyzed, skin surface bacteria collected from the skin surface may be analyzed, or skin color, unevenness, stains, or the like may be analyzed from image information obtained by imaging the user's skin, and a skin classification may be generated in accordance therewith. The proteome and skin surface bacteria are collected using a collection kit, similarly to RNA information. The image of skin is acquired by transmitting photograph data from the user terminal 200 to the cosmetics information providing server 100. One or two or more types of biological information to be analyzed may be used. It is favorable to use one or more types selected from RNA information and proteome (protein) information based on the user's biological tissue, and it is more favorable to use RNA information. Further, information obtained from an image of skin may be used in addition to these pieces of information.

Although cosmetics information has been posted in a ranking format based on the evaluation value on the search result page in the above-mentioned embodiment, the positing format of cosmetics information is not limited thereto. For example, pieces of cosmetics information of a top plurality of highly-evaluated cosmetics may be posted in parallel or text information of a highly-evaluated review in the above qualitative evaluation may be posted together with the cosmetics information.

Further, in the above-mentioned embodiment, cosmetics information has been associated with a skin classification on the basis of evaluation information of a user and cosmetics information has been displayed in association with evaluation information (e.g., as an evaluation ranking) in the search result of cosmetics information. However, cosmetics information may be presented to the user regardless of evaluation information of a user. For example, each piece of cosmetics information may be associated with information indicating to which skin classification of a user each manufacturer recommends the cosmetic.

Further, in this case, the cosmetics information providing server 100 may store sales information of each cosmetic over a predetermined period and return, in response to a search request from a user, which includes a skin classification, a ranking of cosmetics recommended for the skin classification in order of sales to the user terminal 200 as a search result.

In the above-mentioned embodiment, the example in which the user sends the biological sample to the service operator only once and the user is notified of the skin classification generated by the apparatus on the basis of the biological sample only once has been shown. However, the cosmetics information providing server 100 may periodically collect a biological sample from the user, periodically check the skin classification of the user, and notify the user of the skin classification.

That is, the cosmetics information providing server 100 may transmit proposal information for proposing re-provision of a biological sample to the user terminal 200 of the user corresponding to the user ID after a lapse of a predetermined time period from when the skin classification of the user was stored in association with the user ID in the user information database 31. Then, the cosmetics information providing server 100 may store, on the basis of the analysis result of the re-provided biological sample, one of the skin classifications and the user ID again in association with each other, generate time series information regarding the skin classification of the user at the time of previous provision of the biological information and the skin classification of the user at the time of re-provision of the biological information, and transmit the generated time series information to the user terminal of the user.

Here, the predetermined period is, for example, three months, half a year, one year, or the like, but is not limited thereto. Further, the time series information may be information simply indicating the previous skin classification and the current skin classification or may be information further including some kind of advice information regarding skin. Using the time-series skin classification information based on the analysis result of the biological sample, it is possible to provide cosmetics information that suits skin quality for each season to a user whose skin quality differs for each season and provide information indicating that the skin condition has improved or worsened (there has been an effect of use) or has been maintained in a good condition by continuously using the cosmetic for each skin classification. Note that by using analysis information based on biological information such as RNA, it is possible to objectively show the degree of improvement in skin condition.

In the above-mentioned embodiment, the example in which the user who has provided a biological sample and has been notified of his/her skin classification information accesses a search page and searches for cosmetics information according to the skin classification has been shown. However, it is conceivable that there are also users who access the search page without knowing information regarding a skin classification. In this regard, the cosmetics information providing server 100 may display, in the case where the search page is accessed by a user whose biological sample has not been collected (an unloggedin user or a logged-in user whose user ID has not been assigned a skin classification), proposal information for proposing a skin classification based on collection of a biological sample on the search page as, for example, a pop-up or the like. That is, the control unit of the cosmetics information providing server 100 may display, in the case where a search page where a search request can be input is accessed by a user terminal of a user who does not have user identification information associated with a skin classification, information prompting the user to provide biological information for generating the skin classification on the user terminal.

In the above-mentioned embodiment, the cosmetics information providing server 100 has transmitted, to only a user who has provided his/her biological sample, information indicating the skin classification of the user. However, the cosmetics information providing server 100 may transmit information indicating the skin classification of another user. For example, the cosmetics information providing server 100 may transmit information regarding celebrities who have the same skin classification as that of each user to the user terminal 200 when the celebrities have permitted to publish their skin classifications. Further, the cosmetics information providing server 100 may cooperate with the cosmetics information providing service and the SNS (Social networking service) used by each user to post the skin classification of a user who has permitted to publish it as profile information of each user on the SNS. This allows the user to feel close to a celebrity with the same skin classification and consult a friend with the same skin classification about skin concerns.

Although only one cosmetics information providing server 100 has been shown in the above-mentioned embodiment, the processing to be executed by the above cosmetics information providing server 100 may be shared and executed by a plurality of servers. For example, processing of generating a skin classification and processing of providing cosmetics information using the skin classification as a search criterion may be executed by separate servers.

In the above-mentioned embodiment, cosmetics (including external preparations such as quasi-drugs) have been taken as examples of products. The products are favorably cosmetics but are not limited thereto. For example, the product may be beverages, foods, facial beauty devices, or beauty tools, and information regarding these products may be provided to users in accordance with the above skin attributes. Further, biological information regarding the scalp and hair as skin may be analyzed, and the products to be provided to users may be cosmetics for the scalp and hair (including external preparations such as quasi-drugs).

Of the inventions described in the claims of the present application, the invention described as the "information processing method" is one in which each step is automatically performed by at least one apparatus such as a computer through information processing using software and is not one performed by humans using apparatuses such as computers. That is, the "information processing method" is an information processing method using a computer/software, and is not a method in which a calculation tool that is a computer is operated by humans.

The functions realized by the components described in the embodiment of the present invention may be implemented in circuitry or processing circuitry including general-purpose processors, special purpose processors, integrated circuits, ASICs (Application Specific Integrated Circuits), CPUs (Central Processing Units), existing circuits, and/or combinations thereof programed to realize the described functions. Processors include transistors and other circuits and are regarded as circuitry or processing circuitry. Further, processors may be programmed processors that execute a program stored in a memory. In an embodiment of the present invention, circuitry, a unit, and means are hardware that is programmed to realize the described functions or hardware that execute the described functions. The hardware may be any hardware disclosed in the embodiment of the present invention or any hardware programmed to realize the described functions or known to execute the described functions. In the case where the hardware is a processor that is regarded as a type of circuitry, the circuitry, means, or unit is a combination of hardware and software to be used for configuring the hardware and/or processor.

Regarding the above-mentioned embodiment, the present invention further discloses the following information processing system and the like.
<1> An information processing system that provides information regarding a product for skin to each user, including:
   a control unit that
   analyzes pieces of biological information regarding skin of a plurality of users provided from the plurality of users and generates a plurality of skin classifications for classifying the skin of the plurality of users on a basis of an analysis result,
   stores user identification information for identifying a user of a source of the biological information in association with one of the generated skin classifications and stores product information regarding a plurality of products relating to skin in association with the corresponding skin classification,
   receives, from a user terminal of a user corresponding to the user identification information, a search request for product information using the skin classification as a search criterion, and
   searches for the product information corresponding to the skin classification used as the search criterion in accordance with the search request and transmits the searched product information to the user terminal.
<2> The information processing system according to <1>, in which
   the control unit transmits, to a user terminal of the user of the source of the biological information, classification information indicating the skin classification associated with user identification information of the user.
<3> The information processing system according to <2>, in which
   the control unit transmits, to the user terminal, access information for accessing a search page where the search request can be input as well as the classification information.
<4> The information processing system according to <2> or <3>, in which
   the control unit
   receives evaluation information indicating evaluation of each of the users who has used the product on the product,
   stores a skin classification corresponding to user identification information of the user of an evaluation source of the evaluation information, the evaluation information, and the product information of the product that is an evaluation target in the evaluation information in association with each other, and
   transmits, in accordance with the search request, the product information in association with the evaluation information corresponding to the skin classification.
<5> The information processing system according to <4>, in which
   the control unit
   receives attribute information regarding the user who is the source or skin of the user input by the user,
   stores the attribute information in association with the evaluation information,
   receives, from the user terminal, a search request for product information using the skin classification and the attribute information as search criteria, and
   transmits, in accordance with the search request, the product information corresponding to the skin classification in association with the evaluation information corresponding to the attribute information.
<6> The information processing system according to any one of <2> to <5>, in which
   the control unit
   transmits, to the user terminal of the user corresponding to the user identification information, proposal information for proposing re-provision of the biological information after a lapse of a predetermined time period from when the user identification information was stored in association with the skin classification,
   stores, on a basis of an analysis result of the re-provided biological information, the one of the skin classifications and the user identification information in association with each other, and
   generates time series information regarding a skin classification corresponding to the user identification information at a time of previous provision of the biological information and a skin classification corresponding to the user identification information at a time of re-provision of the biological information and transmits the generated time series information to the user terminal corresponding to the user identification information.
<7> The information processing system according to any one of <2> to <6>, in which
   the control unit generates three or more skin classifications on a basis of similarity between data obtained by analyzing the pieces of biological information of the plurality of users regarding two or more evaluation items relating to the biological information.
<8> An information processing method of providing information regarding a product for skin to each user, including:
   analyzing pieces of biological information regarding skin of a plurality of users provided from the plurality of users and generating a plurality of skin classifications for classifying the skin of the plurality of users on a basis of an analysis result:
   storing user identification information for identifying a user of a source of the biological information in association with one of the generated skin classifications and storing product information regarding a plurality of products relating to skin in association with the corresponding skin classification;
   receiving, from a user terminal of a user corresponding to the user identification information, a search request for product information using the skin classification as a search criterion; and
   searching for the product information corresponding to the skin classification used as the search criterion in accordance with the search request and transmits the searched product information to the user terminal.
      <8>
<9> The information processing method according to <8>, further including
   transmitting, to a user terminal of the user of the source of the biological information, classification information indicating the skin classification associated with user identification information of the user.
<10> The information processing method according to <9>, further including
   transmitting, to the user terminal, access information for accessing a search page where the search request can be input as well as the classification information.
<11> The information processing method according to <9> or <10>, further including:
   receiving evaluation information indicating evaluation of each of the users who has used the product on the product;
   storing a skin classification corresponding to user identification information of the user of an evaluation source of the evaluation information, the evaluation information, and the product information of the product that is an evaluation target in the evaluation information in association with each other; and
   transmitting, in accordance with the search request, the product information in association with the evaluation information corresponding to the skin classification.
<12> The information processing method according to <11>, further including:
   receiving attribute information regarding the user who is the source or skin of the user input by the user;
   storing the attribute information in association with the evaluation information;
   receiving, from the user terminal, a search request for product information using the skin classification and the attribute information as search criteria; and
   transmitting, in accordance with the search request, the product information corresponding to the skin classification in association with the evaluation information corresponding to the attribute information.
<13> The information processing method according to any one of <9> to <12>, further including:
   transmitting, to the user terminal of the user corresponding to the user identification information, proposal information for proposing re-provision of the biological information after a lapse of a predetermined time period from when the user identification information was stored in association with the skin classification;
   storing, on a basis of an analysis result of the re-provided biological information, the one of the skin classifications and the user identification information in association with each other; and
   generating time series information regarding a skin classification corresponding to the user identification information at a time of previous provision of the biological information and a skin classification corresponding to the user identification information at a time of re-provision of the biological information and transmits the generated time series information to the user terminal corresponding to the user identification information.
<14> The information processing method according to any one of <8> to <13>, further including
   generating three or more skin classifications on a basis of similarity between data obtained by analyzing the pieces of biological information of the plurality of users regarding two or more evaluation items relating to the biological information.
<15> A program for providing information regarding a product for skin to each user, the program causing an information processing apparatus to execute the steps of:
   analyzing pieces of biological information regarding skin of a plurality of users provided from the plurality of users and generating a plurality of skin classifications for classifying the skin of the plurality of users on a basis of an analysis result:
   storing user identification information for identifying a user of a source of the biological information in association with one of the generated skin classifications and storing product information regarding a plurality of products relating to skin in association with the corresponding skin classification;
   receiving, from a user terminal of a user corresponding to the user identification information, a search request for product information using the skin classification as a search criterion; and
   searching for the product information corresponding to the skin classification used as the search criterion in accordance with the search request and transmits the searched product information to the user terminal.
<16> The program according to <15>, the steps further including
   transmitting, to a user terminal of the user of the source of the biological information, classification information indicating the skin classification associated with user identification information of the user.
<17> The program according to <16>, the steps further including
   transmitting, to the user terminal, access information for accessing a search page where the search request can be input as well as the classification information.
<18> The program according to <16> or <17>, the steps further including:
   receiving evaluation information indicating evaluation of each of the users who has used the product on the product;
   storing a skin classification corresponding to user identification information of the user of an evaluation source of the evaluation information, the evaluation information, and the product information of the product that is an evaluation target in the evaluation information in association with each other; and
   transmitting, in accordance with the search request, the product information in association with the evaluation information corresponding to the skin classification.
<19> The program according to <18>, the steps further including:
   receiving attribute information regarding the user who is the source or skin of the user input by the user;
   storing the attribute information in association with the evaluation information;
   receiving, from the user terminal, a search request for product information using the skin classification and the attribute information as search criteria; and
   transmitting, in accordance with the search request, the product information corresponding to the skin classification in association with the evaluation information corresponding to the attribute information.
<20> The program according to any one of <16> to <19>, the steps further including:
   transmitting, to the user terminal of the user corresponding to the user identification information, proposal information for proposing re-provision of the biological information after a lapse of a predetermined time period from when the user identification information was stored in association with the skin classification;
   storing, on a basis of an analysis result of the re-provided biological information, the one of the skin classifications and the user identification information in association with each other; and
   generating time series information regarding a skin classification corresponding to the user identification information at a time of previous provision of the biological information and a skin classification corresponding to the user identification information at a time of re-provision of the biological information and transmits the generated time series information to the user terminal corresponding to the user identification information.
<21> The program according to any one of <15> to <20>, the steps further including
   generating three or more skin classifications on a basis of similarity between data obtained by analyzing the pieces of biological information of the plurality of users regarding two or more evaluation items relating to the biological information.

## Claims

1. An information processing system that provides information regarding a product for skin to each user, comprising:
a control unit that
analyzes pieces of biological information regarding skin of a plurality of users provided from the plurality of users and generates a plurality of skin classifications for classifying the skin of the plurality of users on a basis of an analysis result,
stores user identification information for identifying a user of a source of the biological information in association with one of the generated skin classifications and stores product information regarding a plurality of products relating to skin in association with the corresponding skin classification,
receives, from a user terminal of a user corresponding to the user identification information, a search request for product information using the skin classification as a search criterion, and
searches for the product information corresponding to the skin classification used as the search criterion in accordance with the search request and transmits the searched product information to the user terminal.

2. The information processing system according to claim 1, wherein
the control unit transmits, to a user terminal of the user of the source of the biological information, classification information indicating the skin classification associated with user identification information of the user.

3. The information processing system according to claim 2, wherein
the control unit transmits, to the user terminal, access information for accessing a search page where the search request can be input as well as the classification information.

4. The information processing system according to claim 2 or 3, wherein
the control unit
receives evaluation information indicating evaluation of each of the users who has used the product on the product,
stores a skin classification corresponding to user identification information of the user of an evaluation source of the evaluation information, the evaluation information, and the product information of the product that is an evaluation target in the evaluation information in association with each other, and
transmits, in accordance with the search request, the product information in association with the evaluation information corresponding to the skin classification.

5. The information processing system according to claim 4, wherein
the control unit
receives attribute information regarding the user who is the source or skin of the user input by the user,
stores the attribute information in association with the evaluation information,
receives, from the user terminal, a search request for product information using the skin classification and the attribute information as search criteria, and
transmits, in accordance with the search request, the product information corresponding to the skin classification in association with the evaluation information corresponding to the attribute information.

6. The information processing system according to any one of claims 2 to 5, wherein
the control unit
transmits, to the user terminal of the user corresponding to the user identification information, proposal information for proposing re-provision of the biological information after a lapse of a predetermined time period from when the user identification information was stored in association with the skin classification,
stores, on a basis of an analysis result of the re-provided biological information, the one of the skin classifications and the user identification information in association with each other, and
generates time series information regarding a skin classification corresponding to the user identification information at a time of previous provision of the biological information and a skin classification corresponding to the user identification information at a time of re-provision of the biological information and transmits the generated time series information to the user terminal corresponding to the user identification information.

7. The information processing system according to any one of claims 1 to 6, wherein
the control unit generates three or more skin classifications on a basis of similarity between data obtained by analyzing the pieces of biological information of the plurality of users regarding two or more evaluation items relating to the biological information.

8. An information processing method of providing information regarding a product for skin to each user, comprising:
analyzing pieces of biological information regarding skin of a plurality of users provided from the plurality of users and generating a plurality of skin classifications for classifying the skin of the plurality of users on a basis of an analysis result:
storing user identification information for identifying a user of a source of the biological information in association with one of the generated skin classifications and storing product information regarding a plurality of products relating to skin in association with the corresponding skin classification;
receiving, from a user terminal of a user corresponding to the user identification information, a search request for product information using the skin classification as a search criterion; and
searching for the product information corresponding to the skin classification used as the search criterion in accordance with the search request and transmits the searched product information to the user terminal.

9. A program for providing information regarding a product for skin to each user, the program causing an information processing apparatus to execute the steps of:
analyzing pieces of biological information regarding skin of a plurality of users provided from the plurality of users and generating a plurality of skin classifications for classifying the skin of the plurality of users on a basis of an analysis result:
storing user identification information for identifying a user of a source of the biological information in association with one of the generated skin classifications and storing product information regarding a plurality of products relating to skin in association with the corresponding skin classification;
receiving, from a user terminal of a user corresponding to the user identification information, a search request for product information using the skin classification as a search criterion; and
searching for the product information corresponding to the skin classification used as the search criterion in accordance with the search request and transmits the searched product information to the user terminal.
